# EUROPÄISCHE PATENTSCHRIFT

(11) **EP 1 699 368 B1**
(45) Veröffentlichungstag und Bekanntmachung des Hinweises auf die Patenterteilung: **08.04.2009**
(21) Anmeldenummer: 05715437.9
(22) Anmeldetag: 22.02.2005
(51) Int. Cl.: A61B 17/32, A61B 17/28

(54) **MEDIZINISCHES SCHNEID UND/ODER HALTEINSTRUMENT**
MEDICAL CUTTING AND/OR HOLDING INSTRUMENT
INSTRUMENT MEDICAL DE COUPE ET/OU DE RETENUE

(30) Priorität: 25.02.2004 DE 102004009199
(43) Veröffentlichungstag der Anmeldung: 13.09.2006
(73) Patentinhaber: Karl Storz GmbH & Co. KG, 78532 Tuttlingen (DE)
(72) Erfinder: STORZ, Martin, 78532 Tuttlingen (DE)
(74) Vertreter: Hofmeister, Frank
(86) Internationale Anmeldenummer: PCT/EP2005/001804
(87) Internationale Veröffentlichungsnummer: WO 2005/079680

(56) Entgegenhaltungen:
- EP-A2- 0 351 540
- WO-A-94/11771
- DE-C- 356 185
- DE-C- 356 185
- DE-C1- 4 313 903
- DE-U1- 9 421 125
- DE-U1- 20 107 029
- DE-U1- 20 107 029
- US-A- 1 754 806
- US-A- 3 404 677
- US-A- 3 404 677
- US-A- 4 369 788
- US-A- 4 369 788
- US-A- 5 312 407
- US-A- 5 312 407
- US-A- 5 665 050
- US-A- 5 665 050

## Beschreibung

Die Erfindung betrifft ein medizinisches Schneid- und/oder Halteinstrument mit einem Schaft, einer am proximalen Ende des Schaftes angeordneten, aus zwei Griffteilen bestehenden Handhabe sowie mit einer Zug-/Schubstange zum Betätigen eines am distalen Ende des Schaftes angeordneten, aus zwei Maulteilen bestehenden Werkzeugs, wobei die Zug-/Schubstange zum Öffnen und Schließen des Werkzeugs distalseitig mit mindestens einem verschwenkbaren Maulteil des Werkzeugs und proximalseitig mit einem verstellbaren Griffteil der Handhabe koppelbar ist.

Medizinische Schneid- und/oder Halteinstrumente werden sowohl in der offenen als auch in der endoskopischen Chirurgie vielfältig eingesetzt. Aufgrund der verwendungsbedingten sehr schlanken Bauweise endoskopischer Instrumente, die in der Regel durch hohle Trokarhülsen in das Operationsgebiet eingeführt werden, haben sich in der endoskopischen Chirurgie sogenannte Rohrschaftinstrumente für die Ausgestaltung von Schneid- und/oder Halteinstrumenten etabliert und bewährt. Diese Rohrschaftinstrumente zeichnen sich dadurch aus, dass der Instrumentenschaft als hohles Rohr ausgebildet ist und die zur Betätigung des distalseitigen Maulteils vorgesehene Zug-/Schubstange verschiebbar im Inneren des hohlen Schaftrohres gelagert ist. Aufgrund dieser konstruktionsbedingten Miniaturisierung der Zug-/Schubstange sowie der beidseitigen Gelenkmechaniken sind derartige Rohrschaftinstrumente nicht für den Einsatz in der Offenen Chirurgie geeignet, in der häufig große Kräfte mittels der Zug-/Schubstange übertragen werden müssen.

Darüber hinaus sind die hohlen Instrumentenschäfte dieser Instrumente nur schwierig zu reinigen und erfordern eine konstruktiv aufwendige Zerlegbarkeit der Instrumente.

Ein gattungsgemäßes, für die Offene Chirurgie geeignetes medizinisches Instrument ist beispielsweise aus der DE 195 13 572 C2 bekannt. Bei diesem bekannten chirurgischen Instrument ist die Zug-/Schubstange in Schlittenführungen gelagert auf dem Instrumentenschaft angeordnet. Diese Konstruktion erlaubt die Verwendung massiver Stangenprofile zur Ausgestaltung des Schaftes sowie der Zug-/Schubstange, so dass auch große Kräfte über die Handhabe auf das distalseitige Werkzeug übertragbar sind.

Nachteilig bei dieser bekannten Konstruktion ist jedoch, dass die Zug-/Schubstange und der Schaft über die Schlittenführungen miteinander gekoppelt sind und somit die beiden Bauteile genau aufeinander abgestimmt sein müssen, was einerseits eine genaue fertigungstechnische Abstimmung der aneinander zu koppelnden Bauteile erfordert und andererseits eine flexible Verwendung des Instruments für andere Einsatzzwecke deutlich erschwert.

DE-C-356 185 Offenbart ein Instrument gemäß dem Oberbegriff des ersten Anspruchs.

Davon ausgehend liegt der Erfindung die **Aufgabe** zugrunde, ein medizinisches Schneid- und/oder Halteinstrument der eingangs genannten Art so auszugestalten, dass dieses bei einfachem konstruktivem Aufbau eine einfache und vielseitige Handhabung ermöglicht.

Die **Lösung** dieser Aufgabenstellung ist erfindungsgemäß dadurch gekennzeichnet, dass der Schaft und die Zug-/Schubstange einander kreuzend angeordnet sind und, dass im Schaft oder in der Zug-/Schubstange ein Durchbruch zum Durchführen des jeweils anderen Bauteils augebildet ist.

Aufgrund der erfindungsgemäßen vollständigen Trennung der Bauteile Schaft und Zug-/Schubstange ist es erstmalig möglich, beide Bauteile unabhängig voneinander genau so auszugestalten, wie es der jeweilige Einsatzzweck erfordert. Neben der einfachen und guten Reinigbarkeit zweier getrennter Bauteile hat diese Ausgestaltungsform den Vorteil, dass der Schaft und/oder die Zug-/Schubstange zur Umrüstung des Instruments einzeln austauschbar sind.

Die Verwendbarkeit eines erfindungsgemäß ausgebildeten medizinischen Instruments für unterschiedliche chirurgische Einsatzzwecke lässt sich gemäß einer praktischen Ausführungsform der Erfindung dadurch erweitern, dass die Zug-/Schubstange und der Schaft in Form und/oder Länge unterschiedlich ausgebildet sind.

Um auch bei engen und vertieften Operationszugängen ein ergonomisch günstiges Arbeiten zu ermöglichen, wird mit der Erfindung weiterhin vorgeschlagen, dass die Zug-/Schubstange und/oder der Schaft mindestens eine Abwinklung oder Krümmung gegenüber der Längsachse aufweisen.

Zur Aufnahme beispielsweise von Lichtleitern und/oder zur Ausbildung von Spül- und Saugkanälen für flüssige und gasförmige Medien ist gemäß einer weiteren Ausführungsform der Erfindung in oder am Schaft und/oder der Zug-/Schubstange mindestens ein in Axialrichtung verlaufender Kanal ausgebildet.

Die Arbeit an tiefliegenden Operationszugängen kann erfindungsgemäß weiterhin dadurch erleichtert werden, dass am Schaft oder an der Zug-/Schubstange ein Beleuchtungselement, beispielsweise mit interner Stromversorgung, festlegbar ist.

Weitere Merkmale und Vorteile der Erfindung ergeben sich anhand der zugehörigen Zeichnung, in der sechs Ausführungsbeispiele eines erfindungsgemäßen medizinischen Schneid- und/oder Halteinstruments nur beispielhaft schematisch dargestellt sind. In der Zeichnung zeigt:

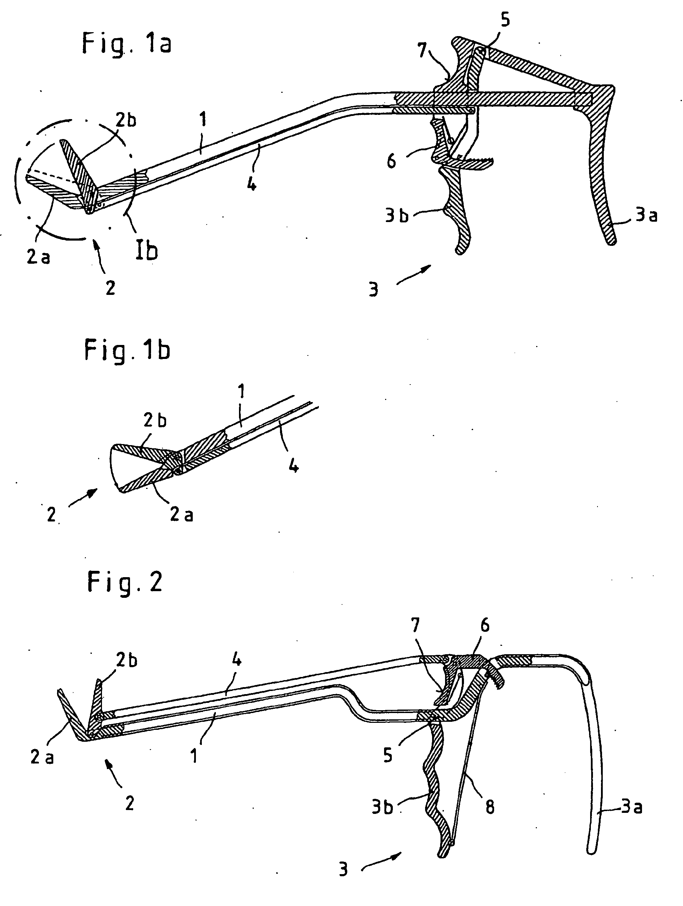

- Fig. 1 a: eine teilweise geschnittene Seitenansicht einer Ausführungsform eines nicht erfindungsgemäßen medizinischen Schneid- und/oder Halteinstruments;
- Fig. 1 b: eine alternative Ausführungsform des Details Ib gemäß Fig. 1 a;
- Fig. 2: eine teilweise geschnittene Seitenansicht einer Ausführungsform eines nicht erfindungsgemäßen medizinischen Schneid- und/oder Halteinstruments;
- Fig. 3: eine teilweise geschnittene Seitenansicht einer Ausführungsform eine nicht erfindungsgemäßen medizinischen Schneid- und/oder Halteinstruments;
- Fig. 4: eine teilweise geschnittene Seitenansicht einer Ausführungsform eines erfindungsgemäßen medizinischen Schneid- und/oder Halteinstruments;
- Fig. 5: eine Seitenansicht einer Ausführungsform eines nicht erfindungsgemäßen medizinischen Schneid- und/oder Halteinstruments und
- Fig. 6: eine teilweise geschnittene Seitenansicht einer Ausführungform eines nicht erfindungsgemäßen medizinischen Schneid- und/oder Halteinstruments.

Die in den Abbildungen Fig. 1a und 2 bis 6 dargestellten medizinischen Schneid- und/oder Halteinstrumente bestehen im Wesentlichen aus einem stangenförmigen Schaft 1, einem am distalen Ende des Schaftes 1 angeordneten, aus zwei Maulteilen 2a und 2b bestehenden Werkzeug 2, einer am proximalen Ende des Schaftes 1 angeordneten, mit zwei Griffteilen 3a und 3b versehenen Handhabe 3 sowie einer axial verschiebbaren Zug-/Schubstange 4 zur Betätigung des Werkzeugs 2 mittels der Handhabe 3.

Wie weiterhin aus den Abbildungen ersichtlich, besteht das Werkzeug 2 aus einem starren, fest mit dem Schaft 1 verbundenen Maulteil 2a sowie einem über die Zug-/Schubstange 4 verschwenkbaren Maulteil 2b. Ebenso weist die Handhabe 3 ein starres, fest mit dem Schaft 1 verbundenes Griffteil 3a sowie ein gegenüber dem starrem Griffteil 3b verstellbares Griffteil 3b auf. Das verschwenkbare Maulteil 2b und das verstellbare Griffteil 3b sind über die Zug-/Schubstange 4 miteinander gekoppelt, wobei die Zug-/Schubstange 4 und das verschwenkbare Griffteil 3b über einen Kopplungsmechanismus lösbar so miteinander verbindbar sind, dass die Zug-/Schubstange 4, beispielsweise zu Reinigungszwecken, nach oben vom Schaft 1 fortgeschwenkt werden kann.

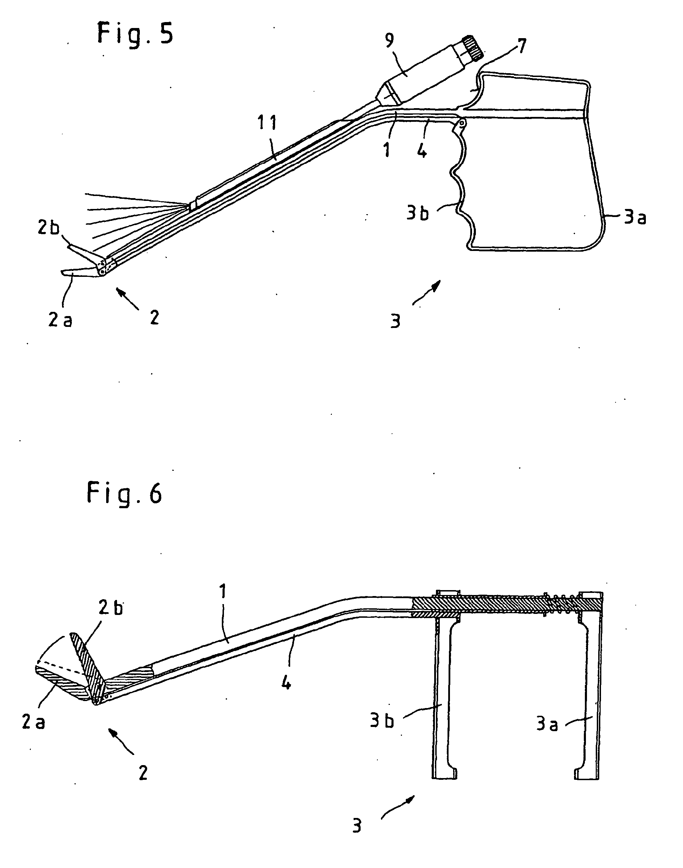

Während bei den in den Abbildungen Fig. 1a bis 4 dargestellten medizinischen Instrumenten das verstellbare Griffteil 3b um eine Schwenkachse 5 gegenüber dem starren Griffteil 3a verschwenkbar ist, ist in den Abbildungen Fig. 5 und 6 eine Ausführungsform dargestellt, bei der das verstellbare Griffteil 3b in Schaftrichtung axial verschiebbar ausgebildet ist.

Durch die Ausbildung der Zug-/Schubstange 4 als eigenständiges und unabhängig von der Ausgestaltung des Schaftes 1 ausgebildetes Bauteil, das außerhalb der Schaftes 1 angeordnet, ausschließlich am verschwenkbaren Maulteil 2b des Werkzeugs 2 und am verstellbaren Griffteil 3b der Handhabe 3 gelagert ist, können beide Bauteile 1 und 4 individuell und an den jeweiligen Einsatzzweck angepasst ausgestaltet werden. Auch können beide Bauteile 1 und 4, wie für die Verwendung in der Offenen Chirurgie erforderlich, als massive Stangen ausgebildet werden, über die sich auch hohe Kräfte von der Handhabe 3 auf das Werkzeug 2 übertragen lassen.

Neben der unterschiedlichen Art zur Verlagerung des verstellbaren Griffteils 3b der Handhabe 3, nämlich als verschwenkbares Griffteil 3b gemäß Fig. 1a bis 4 oder als axial verschiebbares Griffteil 3b gemäß Fig. 5 und 6, unterscheiden sich die in den Abbildungen Fig. 1a bis 6 dargestellten Ausführungsformen medizinischer Schneid- und/oder Halteinstrumente im Wesentlichen durch die form- und längenmäßige Ausgestaltung der Schäfte 1 und Zug-/Schubstangen 4.

Bei der in Fig. 1a dargestellten ersten Ausführungsform verlaufen die Zug-/Schubstange 4 und der Schaft 1 jeweils mit einer Abwinklung nach unten versehen parallel zueinander, wobei die Zug-/Schubstange 4 bei dieser Ausführungsform unterhalb des Schaftes 1 angeordnet ist.

Das um die am starren Griffteil 3a angeordnete Schwenkachse 5 verschwenkbare Griffteil 3b ist über eine vorzugsweise federbelastete Arretiervorrichtung 6 in der gegenüber dem starren Griffteil 3a verschwenkten Stellung fixierbar. Wie weiterhin aus Fig. 1a ersichtlich, ist am starren Griffteil 3a der Handhabe 3 eine Auflage 7 für den Zeigefinger des Benutzers des medizinischen Instruments ausgebildet, um den üblicherweise in der Offenen Chirurgie bei der Betätigung des verstellbaren Griffteils 3b erzeugten Schaftausschlag kompensieren zu können. Diese vom verschwenkbaren Griffteil 3b unabhängige Zeigefingerauflage 7 erlaubt dem Benutzer somit zusammen mit der Abstützung der Hand am starren Griffteil 3a der Handhabe 3 eine ruhige und lagestabilisierte Betätigung des medizinischen Instruments.

Die in den Abbildungen Fig. 1a und 1b dargestellten Ausführungsformen unterscheiden sich durch die Ausrichtung der Maulteile 2a und 2b des Werkzeugs 2 zur Längsachse des Schaftes 1 sowie der Zug-/Schubstange 4. Während bei der in Fig. 1a dargestellten Ausführungsform die Maulteile 2a und 2b des Werkzeugs 2 deutlich gegenüber der Längsachse abgewinkelt ausgebildet sind, verlaufen sie bei der in Fig. 1b dargestellten Ausführungsform im Wesentlichen in Achsrichtung.

Bei der in Fig. 2 dargestellten zweiten Ausführungsform verlaufen die Zug-/Schubstange 4 und der Schaft 1 nur teilweise parallel zueinander, wobei die Zug-/Schubstange 4 bei dieser Ausführungsform oberhalb des Schaftes 1 angeordnet ist.

Das um die am Schaft 1 angeordnete Schwenkachse 5 verschwenkbare Griffteil 3b ist wiederum über eine federbelastete Arretiervorrichtung 6 in der gegenüber dem starren Griffteil 3a verschwenkten Stellung fixierbar. Wie weiterhin aus Fig. 2 ersichtlich, sind bei dieser Ausführungsform die Auflage 7 für den Zeigefinger des Benutzers und die Arretiervorrichtung 6 einstückig so ausgebildet, dass bei aufliegendem Zeigefinger die Arretiervorrichtung 6 deaktiviert ist, beim Freigeben der Zeigefingerauflage 7 die Arretiervorrichtung 6 aber aufgrund der Federbelastung sofort selbsttätig sperrend am starrem Griffteil 3a eingreift. Um Platz für die Zeigefingerauflage 7 zu schaffen, ist der Schaft 1 an dieser Stelle nach unten von der Zug-/Schubstange 4 fort abgewinkelt ausgebildet.

Weiterhin weist die Handhabe 3 ein Federelement 8 auf , über das das verschwenkbare Griffteil 3b in die das Werkzeug 2 öffnende Stellung vorgespannt ist.

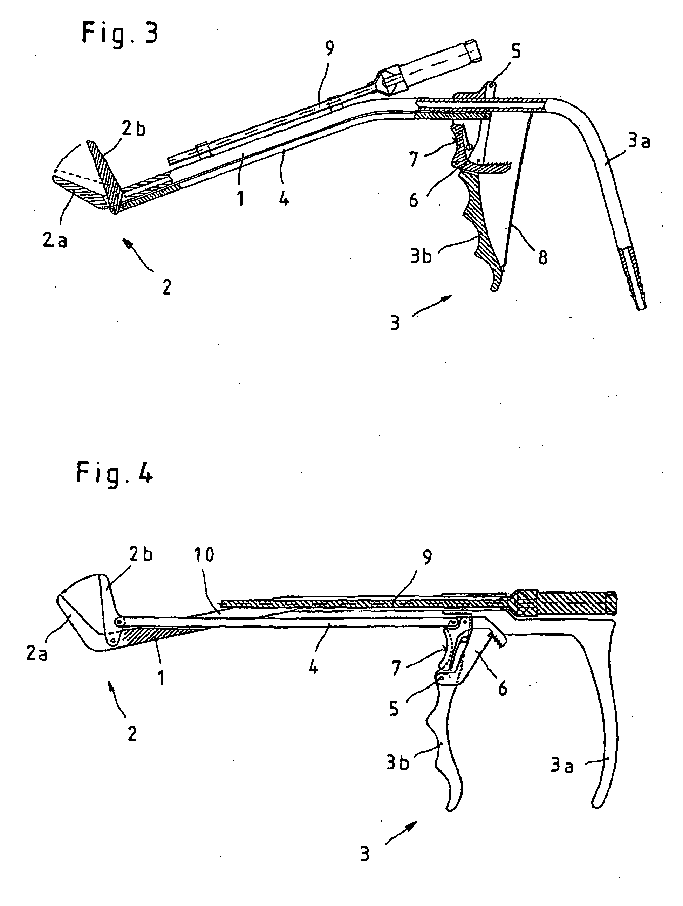

Bei der in Fig. 3 dargestellten Ausführungsform verlaufen die Zug-/Schubstange 4 und der Schaft 1, wie bereits zur Fig. 1a beschrieben, jeweils mit einer Abwinklung nach unten versehen parallel zueinander, wobei die Zug-/Schubstange 4 unterhalb des Schaftes 1 angeordnet ist.

Das um die am Schaft 1 angeordnete Schwenkachse 5 verschwenkbare und über das Federelement 8 in Öffnungsrichtung des Werkzeugs 2 vorgespannte Griffteil 3b ist auch bei dieser Ausführungsform über eine federbelastete Arretiervorrichtung 6 in der gegenüber dem starren Griffteil 3a verschwenkten Stellung fixierbar, wobei die Auflage 7 für den Zeigefinger des Benutzers und die Arretiervorrichtung 6 einstückig so ausgebildet, wie dies zuvor zur zweiten Ausführungsform gemäß Fig. 2 beschrieben wurde.

Die in den Abbildungen Fig. 2 und 3 dargestellten Instrumente zeichnen sich weiterhin dadurch aus, dass das starre Griffteil 3a und der Schaft 1 als einstückiges Bauteil ausgebildet sind, wodurch sich die Montage und Demontage des Instruments deutlich vereinfachen lassen.

Um für den Operateur die Sichtverhältnisse bei engen Operationszugängen zu verbessern, ist am Schaft 1 ein Beleuchtungselement 9 festlegbar ist, das vorzugsweise mittels Batterie oder Akku über eine eigene Stromversorgung verfügt. Neben der in Fig. 3 dargestellten Ausführungsform, bei der das Beleuchtungselement 9 am Schaft1 festlegbar ist, ist es selbstverständlich auch möglich, das Beleuchtungselement 9 an der Zug-/Schubstange 4 anzuordnen, insbesondere, wenn diese, wie in Fig. 2 dargestellt, oberhalb des Schaftes 1 angeordnet ist.

Bei der in Fig. 4 dargestellten Erfindungsgemäßen Ausführungsform kreuzen sich der Verlauf der geraden Zug-/Schubstange 4 und des einfach nach unten abgewinkelten Schaftes 1. Zu dieser Ausgestaltung ist im Schaft 1 ein Durchbruch 10 ausgebildet, durch den die Zug-/Schubstange 4 durch den Schaft 1 hindurchtritt, so dass die Zug-/Schubstange 4 unterhalb des Schaftes 1 am verschwenkbaren Griffteil 3b gelagert ist, aber oberhalb des Schaftes 1 am verschwenkbaren Maulteil 2b gelagert ist.

Das um die am starren Griffteil 3a angeordnete Schwenkachse 5 verschwenkbare Griffteil 3b ist auch bei dieser Ausführungsform über eine federbelastete Arretiervorrichtung 6 in der gegenüber dem starren Griffteil 3a verschwenkten Stellung fixierbar, wobei die Auflage 7 für den Zeigefinger des Benutzers und die Arretiervorrichtung 6 einstückig so ausgebildet, wie dies zuvor zur zweiten Ausführungsform gemäß Fig. 2 beschrieben wurde.

Auch diese dargestellte Ausführungsform zeigt die Verwendung eines am Instrument festlegbaren Beleuchtungselements 9. Auch wenn nicht alle dargestellten medizinischen Instrumente mit einem Beleuchtungselement 9 ausgestattet sind, besteht bei allen Instrumenten die Möglichkeit, diese mit einem derartigen Beleuchtungselement 9 auszurüsten, wenn dies erforderlich ist.

Die in Fig. 5 und 6 dargestellten Ausführungsformen zeigen schließlich ein medizinisches Schneid- und/oder Halteinstrument mit einem axial verschiebbaren Griffteil 3b.

Bei diesen beiden Ausführungsformen verlaufen die Zug-/Schubstangen 4 und die Schäfte 1 jeweils mit einer Abwinklung nach unten versehen parallel zueinander, wobei die Zug-/Schubstange 4 jeweils unterhalb des Schaftes 1 angeordnet ist. Selbstverständlich ist es aber auch bei dieser Art der Ausgestaltung des verstellbaren Griffteils 3b möglich, den konstruktiven Aufbau so auszugestalten, dass die Zug-/Schubstange 4 jeweils oberhalb des Schaftes 1 angeordnet ist.

Die in Fig. 5 dargestellte Ausführungsform zeichnet sich ferner dadurch aus, dass das axial verschiebbare Griffteil 3b einstückig mit dem starren Griffteil 3a ausgebildet ist und die Verstellbarkeit des Griffteils 3b auf der Federelastizität des Handhabenmaterials beruht.

Zur Aufnahme des Beleuchtungselements 9 weist das in Fig. 5 dargestellte medizinische Instrument auf der Oberseite des Schaftes 1 einen Kanal 11 auf, in das Beleuchtungselement 9 einsetzbar ist.

Die wie dargestellt ausgebildeten medizinischen Schneid- und/oder Halteinstrumente zeichnen sich dadurch aus, dass die unabhängig vom Schaft 1 ausgebildeten Zug-/Schubstangen 4 einerseits einfach und schnell zu reinigen und zu montieren sind und andererseits die Schäfte 1 und Zug-/Schubstangen 4 individuell an den jeweiligen Einsatzzweck anpassbar sind. Die solchermaßen ausgebildeten medizinische Instrumente sind aufgrund des konstruktiv einfachen robusten Aufbaus gut für den Einsatz in der Offenen Chirurgie geeignet.

### Bezugszeichenliste

- 1: Schaft
- 2: Werkzeug
- 2a: starres Maulteil
- 2b: verschwenkbares Maulteil
- 3: Handhabe
- 3a: starres Griffteil
- 3b: verstellbares Griffteil
- 4: Zug-/Schubstange
- 5: Schwenkachse
- 6: Arretiervorrichtung
- 7: Auflage
- 8: Federelement
- 9: Beleuchtungselement
- 10: Durchbruch
- 11: Kanal

## Patentansprüche

1. Medizinisches Schneid- und/oder Halteinstrument mit einem Schaft (1), einer am proximalen Ende des Schaftes (1) angeordneten, aus zwei Griffteilen (3a, 3b) bestehenden Handhabe (3) sowie mit einer Zug-/Schubstange (4) zum Betätigen eines am distalen Ende des Schaftes (1) angeordneten, aus zwei Maulteilen (2a, 2b) bestehenden Werkzeugs (2), wobei die Zug-/Schubstange (4) zum Öffnen und Schließen des Werkzeugs (2) distalseitig mit mindestens einem verschwenkbaren Maulteil (2b) des Werkzeugs (2) und proximalseitig mit einem verstellbaren Griffteil (3b) der Handhabe (3) koppelbar ist und wobei die Zug-/Schubstange (4) als vom Schaft (1) unabhängiges Bauteil ausgebildet ist, das außerhalb der Schaftes (1) angeordnet, ausschließlich am verschwenkbaren Maulteil (2b) des Werkzeugs (2) und am verstellbaren Griffteil (3b) der Handhabe (3) gelagert ist,
**dadurch gekennzeichnet,**
**dass** der Schaft (1) und die Zug-/Schubstange (4) einander kreuzend angeordnet sind und, dass im Schaft (1) oder in der Zug-/Schubstange (4) ein Durchbruch (10) zum Durchführen des jeweils anderen Bauteils (4 oder 1) ausgebildet ist.

2. Medizinisches Schneid- und/oder Halteinstrument nach Anspruch 1, **dadurch gekennzeichnet, dass** die Zug-/Schubstange (4) und der Schaft (1) in Form und/oder Länge unterschiedlich ausgebildet sind.

3. Medizinisches Schneid- und/oder Halteinstrument nach Anspruch 1 oder 2, **dadurch gekennzeichnet, dass** die Zug-/Schubstange (4) und/oder der Schaft (1) mindestens eine Abwinklung gegenüber der Längsachse aufweisen.

4. Medizinisches Schneid- und/oder Halteinstrument nach einem der Ansprüche 1 bis 3, **dadurch gekennzeichnet, dass** in oder am Schaft (1) und/oder der Zug-/Schubstange (4) mindestens ein in Axialrichtung verlaufender Kanal (11) ausgebildet ist.

5. Medizinisches Schneid- und/oder Halteinstrument nach einem der Ansprüche 1 bis 4, **dadurch gekennzeichnet, dass** am Schaft (1) oder an der Zug-/Schubstange (4) ein Beleuchtungselement (9) festlegbar ist.

## Claims

1. Medical cutting and/or holding instrument comprising a shaft (1), a handle (3) arranged at the proximal end of the shaft (1) and consisting of two grip parts (3a, 3b), and a pull/push rod (4) for actuating a tool (2) arranged at the distal end of the shaft (1) and consisting of two jaw parts (2a, 2b), wherein, for opening and closing the tool (2), the pull/push rod (4) can be coupled at the distal end to at least one pivotable jaw part (2b) of the tool (2) and can be coupled at the proximal end to an adjustable grip part (3b) of the handle (3) and wherein the pull/push rod (4) is formed as a component, which is independent of the shaft (1), arranged outside the shaft (1), and which is solely mounted on the pivotable jaw part (2b) of the tool (2) and on the adjustable grip part (3b) of the handle (3), **characterized in that** the shaft (1) and the pull/push rod (4) are arranged crossing one another and **in that** an opening (10) is formed in the shaft (1) or in the pull/push rod (4) for leading through the other component (4 or 1) respectively.

2. Medical cutting and/or holding instrument according to Claim 1, **characterized in that** the pull/push rod (4) and the shaft (1) are formed differently in shape and/or length.

3. Medical cutting and/or holding instrument according to Claim 1 or 2, **characterized in that** the pull/push rod (4) and/or the shaft (1) have at least one angled portion with respect to the longitudinal axis.

4. Medical cutting and/or holding instrument according to one of Claims 1 to 3, **characterized in that** at least one channel (11) running in the axial direction is formed in or on the shaft (1) and/or the pull/push rod (4).

5. Medical cutting and/or holding instrument according to one of Claims 1 to 4, **characterized in that** a lighting element (9) can be fixed to the shaft (1) or to the pull/push rod (4).

## Revendications

1. Instrument médical de coupe et/ou de maintien, comportant une tige (1), une poignée (3) disposée à l'extrémité proximale de la tige (1), constituée de deux parties branches (3a, 3b), ainsi qu'une barre de traction/poussée (4) destinée à actionner un outil disposé à l'extrémité distale de la tige (1), constitué de deux parties mors (2a, 2b), étant entendu que la barre de traction/poussée (4), en vue de l'ouverture et de la fermeture de l'outil (2), peut être couplée du côté distal à au moins une partie mors pivotable (2b) de l'outil (2) et du côté proximal à une partie branche réglable (3b) de la poignée (3) et que la barre de traction/poussée (4) est conçue en tant que module indépendant de la tige (1), disposé hors de la tige (1) et logé exclusivement sur la partie mors pivotable (2b) de l'outil (2) et sur la partie branche (3b) réglable de la poignée (3),
**caractérisé en ce que**
la tige (1) et la barre de traction/poussée (4) sont disposées en croix et qu'il est prévu dans la tige (1) ou dans la barre de traction/poussée (4) une ouverture (10) pour permettre le passage de l'autre module (4) ou (1) considéré.

2. Instrument médical de coupe et/ou de maintien selon la revendication 1, **caractérisé en ce que** la barre de traction/poussée (4) et la tige (1) sont conçues de façon à être de forme et/ou de longueur différentes.

3. Instrument médical de coupe et/ou de maintien selon la revendication 1 ou 2, **caractérisé en ce que** la barre de traction/poussée (4) et/ou la tige (1) présentent au moins une déflexion par rapport à l'axe longitudinal.

4. Instrument médical de coupe et/ou de maintien selon une des revendications 1 à 3, **caractérisé en ce qu'**il est prévu au moins un canal (11) s'étendant en direction axiale dans ou sur la tige (1) et/ou la barre de traction/poussée (4).

5. Instrument médical de coupe et/ou de maintien selon une des revendications 1 à 4, **caractérisé en ce qu'**un élément d'éclairage (9) peut être fixé sur la tige (1) ou sur la barre de traction/poussée (4).
